# EUROPÄISCHE PATENTSCHRIFT

(11) **EP 2 561 800 B1**
(45) Veröffentlichungstag und Bekanntmachung des Hinweises auf die Patenterteilung: **28.12.2016**
(21) Anmeldenummer: 12180854.7
(22) Anmeldetag: 17.08.2012
(51) Int. Cl.: A61B 3/107

(54) **Ophthalmologisches Analysegerät und Verfahren**
Ophthalmological analysis apparatus and method
Appareil d'analyse ophtalmologique et procédé

(30) Priorität: 26.08.2011 DE 102011081642; 12.09.2011 DE 102011082500
(43) Veröffentlichungstag der Anmeldung: 27.02.2013
(73) Patentinhaber: Oculus Optikgeräte GmbH, 35582 Wetzlar (DE)
(72) Erfinder: Steinmüller, Andreas, 35435 Wettenberg (DE)
(74) Vertreter: advotec.

(56) Entgegenhaltungen:
- US-A- 5 838 811
- US-B2- 7 264 355

## Beschreibung

Die Erfindung betrifft ein ophthalmologisches Analysegerät mit den Merkmalen des Anspruchs 1 sowie ein Analyseverfahren mit den Merkmalen des Anspruchs 8.

Ophthalmologische Analysegeräte zur Bestimmung einer Krümmung einer Cornea sind hinreichend bekannt. Diese Analysegeräte umfassen regelmäßig eine Projektionseinrichtung mittels der ein Bildmuster auf eine Cornea eines Auges projiziert werden kann. Das Bildmuster kann beispielsweise ein punktartiges Bildmuster oder auch in Form eines Rings bei einem Keratometer ausgebildet sein. Insbesondere bei einem Videokeratometer wird ein Bildmuster konzentrischer Ringe, sogenannte Placidoringe, verwandt. Diese Bildmuster werden regelmäßig mittels einer in einer optischen Achse des Auges ausgerichteten Kamera aufgenommen, wobei aus den so erfassten Bildern der Cornea mit einer Abbildung des betreffenden Bildmusters eine Krümmung der Cornea errechnet werden kann. Neben einer Untersuchung einer Cornea, beispielsweise hinsichtlich eines Keratokonus, werden Keratometer auch für eine Auswahl und Anpassung von Kontaktlinsen verwendet. Weiter ist es bekannt, ein Keratometer, welches eine erste Analysevorrichtung ausbildet, mit einer zweiten Analysevorrichtung zu ergänzen. Die zweite Analysevorrichtung kann beispielsweise ein sogenanntes Pachymeter sein, mit dem eine Messung einer Dicke der Cornea ermöglicht wird. Das Pachymeter bzw. die zweite Analysevorrichtung umfasst dann regelmäßig eine weitere Projektionseinrichtung, mit der ein Lichtspalt auf eine Oberfläche des Auges projiziert werden kann. Mittels einer Beobachtungseinrichtung wird der Lichtspalt bzw. ein durch den Lichtspalt erzeugtes Schnittbild der Cornea aufgenommen. Aus diesem Bild des Lichtspalts kann dann eine Dicke der Cornea errechnet werden. Auch ist es möglich anhand des Schnittbilds, wie auch mit dem Keratometer bzw. der ersten Analysevorrichtung einen Krümmungsradius der Cornea zu bestimmen.

Bei den bekannten Analysegeräten bzw. Analyseverfahren ist es nachteilig, dass immer nur ein Zentralradius der Cornea bestimmt werden kann. So ist es mittels eines Pachymeters nicht möglich einen Krümmungsradius einer Sclera zu messen, da hier mangels optisch transparenten Gewerbematerials kein Schnittbild erzeugt werden kann. Auch werden für diese Messungen regelmäßig Spaltlampen verwendet, deren Spaltbreite auf eine Größe der Cornea reduziert ist. Eine verlässliche Messung der Sclera bzw. eines Limbus ist mit den bekannten Keratometern kaum möglich, da diese als Bildmuster konzentrische Ringe verwenden, die einen Krümmungssprung im Bereich des Limbus nicht erfassen können. Auch sind die bekannten Keratometer primär auf eine Messung der Cornea ausgelegt, die im Wesentlichen einen konstanten Krümmungsradius aufweist.

Zur Anpassung weicher Kontaktlinsen ist regelmäßig eine Messung eines Zentralradius der Cornea erforderlich. Jedoch wird mit den vorbeschriebenen, bekannten Messverfahren keine Tiefe der Cornea relativ zu einem Apex des Auges bzw. eine Grundform des Auges einschließlich Limbus und Sclera ermittelt. Diese Grundform kann jedoch für eine Anpassung einer weichen Kontaktlinse wesentlich sein. Bei einer Abweichung eines cornealen Krümmungsradius in der Nähe der Sclera können unerwünschte Spannungen in der Cornea sowie der betreffenden Kontaktlinse entstehen. Sofern eine Form der Kontaktlinse nicht einer Form der Cornea folgt, übt die Kontaktlinse eine unerwünschte Kraft auf die Cornea aus und kann diese verformen. Auch kann ein äußerer Rand der Kontaktlinse infolge eines veränderten Krümmungsradius beispielsweise aufgeweitet werden bzw. der Rand der Kontaktlinse in den Bereich des Limbus hineinreichen. Bei einer Anpassung einer Kontaktlinse werden derartige Probleme häufig nicht bemerkt, und können erst durch weitere, gesonderte Messungen des betreffenden Auges festgestellt und quantifiziert werden.

Aus der US 7,264,355 ist ein ophthalmologisches Analysegerät bekannt, welches eine erste Analysevorrichtung aufweist, die ausgebildet ist, eine Krümmung einer Cornea eines Auges zu messen. Das Analysegerät weist des Weiteren eine zweite Analysevorrichtung auf, welche einen Lichtprojektor und Beobachtungseinrichtungen umfasst. Mittels des Lichtprojektors wird ein Lichtspalt auf eine Oberfläche des Auges projiziert, wobei mittels einer Beobachtungseinrichtung ein Abbild des Lichtspalts aufgenommen wird. Mit dem Lichtprojektor kann der Lichtspalt auf einen Bereich eines Limbus des Auges projiziert werden.

Die US 5,838,811 beschreibt ein Analysegerät mit einer einzelnen Spaltprojektionseinheit zur Projektion eines Lichtspalts auf eine Oberfläche eines Auges. Weiter kann mit einer Kamera eine vordere Oberfläche der Cornea und damit ein Abbild des auf der Oberfläche auftreffenden Lichtspalts aufgenommen werden. Dabei kann eine Topografie aus dem Abbild auf Basis einer Bestimmung von Koordinaten mittels einer CAD-Software berechnet werden. Insbesondere ist auch vorgesehen, sogenannte Limbuspunkte vor einer Berechnung der Topografie mit der CAD-Software zu entfernen. Das Analysegerät erfordert eine Kontaktlinse zur Kalibrierung, die den der Cornea zugehörigen Bereich des Auges abdeckt.

Der vorliegenden Erfindung liegt daher die Aufgabe zugrunde, ein ophthalmologisches Analysegerät sowie ein Analyseverfahren vorzuschlagen, dass eine genauere Anpassung einer Kontaktlinse an ein Auge ermöglicht.

Diese Aufgabe wird durch eine Vorrichtung mit den Merkmalen des Anspruchs 1 und ein Verfahren mit den Merkmalen des Anspruchs 8 gelöst.

Das erfindungsgemäße ophthalmologische Analysegerät weist die Merkmale des Anspruchs 1 auf.

Demnach ist das Analysegerät aus zwei Analysevorrichtungen gebildet, wobei die zweite Analysevorrichtung insbesondere zur Messung eines Profils in einem Übergangsbereich von einer Cornea zu einer Sclera dient. Die Kenntnisse des betreffenden Profils im Bereich des Limbus können vorteilhaft zur Anpassung einer weichen Kontaktlinse verwendet werden, da so festgestellt werden kann, ob durch eventuell abweichende Krümmungsradien in einem Randbereich der Cornea durch die Kontaktlinse Spannungen in der Cornea indiziert werden bzw. die Kontaktlinse eine Kraft auf die Cornea ausübt oder gegebenenfalls die Kontaktlinse bis in den Bereich des Limbus hineinragt. Die zweite Analysevorrichtung kann hier besonders einfach ausgebildet werden, da sie lediglich aus einer Spaltprojektionseinheit und einer zugehörigen Beobachtungseinrichtung gebildet ist, mit der der in dem Bereich des Limbus sichtbare Lichtspalt erfasst wird. So wird es möglich eine Lage des Limbus relativ zum Apex bzw. der Cornea sehr genau zu bestimmen und Krümmungsradien der Cornea und der Sclera sowie des Limbus aus dem aufgenommenen Bild abzuleiten bzw. zu errechnen.

Bei der zweiten Analysevorrichtung kann ein Strahlengang bzw. eine Geräteachse der Beobachtungseinrichtung in einer Richtung einer optischen Achse des Auges ausrichtbar sein, wobei ein Projektionsstrahlengang der Spaltprojektionseinheit relativ zum Strahlengang der Beobachtungseinrichtung in einem Winkel α angeordnet sein kann. Die Beobachtungseinrichtung der zweiten Analysevorrichtung kann demnach so angeordnet sein, dass sie in Richtung der optischen Achse des Auges ausgerichtet werden kann. Die Spaltprojektionseinheit kann in diesem Fall zum Projektionsstrahlengang zur Projektion des Lichtspalts auf das Auge seitlich schräg zum Strahlengang der Beobachtungseinrichtung angeordnet sein. Der Projektionsstrahlengang kann so zu einer Sagittalebene des Strahlengangs der Beobachtungseinrichtung in einem Winkel α ausgerichtet sein. Somit wird das Bild des Lichtspalts auf der Cornea und der Sclera nicht geradförmig sondern an einen jeweiligen Krümmungsradius derselben angepasst aus Richtung der Beobachtungseinrichtung sichtbar. Da der Winkel α bekannt ist, kann aus der ermittelten Verzerrung des Lichtspalts der jeweilige Krümmungsradius leicht errechnet werden.

Erfindungsgemäß kann ein Strahlengang bzw. eine Geräteachse der Beobachtungseinrichtung relativ zu einem Projektionsstrahlengang bzw. einer Spaltprojektionsebene der Spaltprojektionseinheit in einem Winkel α angeordnet sein, wobei die Spaltprojektionsebene der Spaltprojektionseinheit in einer Richtung zu einer optischen Achse des Auges ausrichtbar ist. Demnach kann die Spaltprojektionseinheit so auf das Auge ausgerichtet sein bzw. das Auge auf die Spaltprojektionseinheit, dass der Lichtspalt auf der Cornea und der Sclera aus Richtung der Spaltprojektionseinheit geradförmig erscheint. Hierbei wird es möglich, eine Horizontalebene bzw. Mittenebene des Auges mit dem Lichtspalt unmittelbar zu beleuchten. Zwar erscheint der Lichtspalt dann aus Richtung der Beobachtungseinrichtung ebenfalls nicht geradförmig, jedoch können die jeweiligen Krümmungsradien dann noch einfacher errechnet werden, da der Lichtspalt mit dem Apex in der Horizontalebene des Auges liegt. Folglich kann demnach eine Sehachse des Auges in dem Winkel α gegenüber dem Strahlengang bzw. der Geräteachse der Beobachtungseinrichtung geneigt sein. So kann das Auge nach unten geneigt sein, was einer natürlichen Sehachsenausrichtung entspricht.

Weiter kann der Projektionsstrahlengang der Spaltprojektionseinheit relativ zu der Sagittalebene in dem Winkel α und in einem Winkel β zu einer Meridionalebene des Strahlengangs der Beobachtungseinrichtung angeordnet sein. Alternativ ist es natürlich auch möglich den Projektionsstrahlengang parallel zu der Meridionalebene auszurichten. Eine Ausrichtung des Projektionsstrahlengangs mit den Winkeln α und β ermöglicht eine flexiblere Positionierung der Spaltprojektionseinheit wenn beispielsweise als erste Analysevorrichtung ein Keratometer mit Placidoringen verwendet wird. Eine auf Placidoringen basierende Projektionseinrichtung erfordert einen besonders großen Bauraum, so dass diese bei einer zu dem Strahlengang der Beobachtungseinrichtung parallelen Anordnung der Spaltprojektionseinheit bzw. des betreffenden Projektionsstrahlengangs hinderlich sein kann.

Vorteilhaft kann ein Durchlass in einer weiteren Projektionseinrichtung der ersten Analysevorrichtung für den Strahlengang der Beobachtungseinrichtung ausgebildet sein. Insofern es sich bei der weiteren Projektionseinrichtung der ersten Analysevorrichtung um eine Placido-Projektionseinrichtung handelt, kann dann die Beobachtungseinrichtung von einem Auge aus gesehen hinter der weiteren Projektionseinrichtung angeordnet werden, wodurch das erste und das zweite Analysegerät gleichzeitig verwendet werden können.

Erfindungsgemäß umfasst die Beobachtungseinrichtung der zweiten Analysevorrichtung eine Kamera. Wobei die Beobachtungseinrichtung eine Kamera mit einem Objektiv umfasst, wobei die Beobachtungseinrichtung auch als eine Beobachtungseinrichtung der ersten Analysevorrichtung dienen kann. Wenn die erste Analysevorrichtung ein Keratometer ist, kann dann mit der Beobachtungseinrichtung ein Bildmuster einer weiteren Projektionseinrichtung des Keratometers aufgenommen und gleichzeitig das Bild des Lichtspalts des Spaltprojektionseinheit der zweiten Analysevorrichtung erfasst werden. Das Analysegerät wird so besonders kostengünstig ausbildbar, da lediglich eine Kamera mit einem Objektiv für beide Analysevorrichtungen benötigt wird. Dann kann die Beobachtungseinrichtung gleichzeitig zur Messung der Topografie der Cornea und zur Bestimmung der Topografie der Oberfläche des Auges im Bereich des Limbus verwendet werden.

Weiter kann die erste Analysevorrichtung ein Videokeratometer sein, beispielsweise mit einer Placido-Projektionseinrichtung, wobei dann die erste Analysevorrichtung und die zweite Analysevorrichtung in einem gemeinsamen Gehäuse des Analysegerätes angeordnet sein können. Erfindungsgemäß weist die Projektionseinrichtung zwei Spaltprojektionseinheiten auf. Mit zwei Spaltprojektionseinheiten wird es möglich, eine Topografie in zwei unterschiedlichen Bereichen des Limbus gleichzeitig zu ermitteln. So können auch besonders einfach unmittelbar verfügbare und besonders genaue Messdaten eines Durchmessers d des Limbus bzw. der Cornea gewonnen werden. Es ist daher nicht einmal notwendig eine Spaltprojektionseinheit zur aufeinanderfolgenden Messung der beiden Bereiche gegebenenfalls zu verschwenken.

Die Spaltprojektionseinheiten können dann auch relativ zu einer Geräteachse der Beobachtungseinrichtung symmetrisch angeordnet sein. Der Durchmesser d des Limbus bzw. der Cornea ist dann besonders genau bestimmbar.

Erfindungsgemäß weist das Analysegerät eine Auswertevorrichtung auf, mittels der das Bild analysiert werden kann. Im Übrigen kann das Analysegerät eine Auswertevorrichtung für beide Analysevorrichtungen aufweisen, mittels der das Bild analysiert werden kann. So kann eine weitere Reduzierung der Herstellungskosten für das Analysegerät erzielt werden. Weiter können die mit den beiden Analysevorrichtungen gewonnen Messdaten in sinnvoller Weise verknüpft werden. Vorteilhaft kann die Auswertevorrichtung im Analysegerät selbst angeordnet sein und eine Verarbeitung der Bilder sowie eine visualisierte Ausgabe der von der Auswertevorrichtung ermittelten Messergebnisse ermöglichen. Die Auswertevorrichtung kann insbesondere Mittel zur Datenverarbeitung umfassen, die dann auch eine digitale Verarbeitung der Bilder durchführen. Auch ist es denkbar, dass die Mittel zur Datenverarbeitung Datenspeicher mit einer Datenbank aufweisen, wobei die Datenbank Vergleichsdatensätze von Bildern oder Messparametern aufweisen kann. Aus derartigen Vergleichsdatensätzen können dann, beispielsweise durch einen Bildvergleich, vereinfachte Rückschlüsse auf wahrscheinliche Messergebnisse oder auch Korrekturen von Messergebnissen durchgeführt werden. Eine Auswertung kann dadurch wesentlich beschleunigt und eine Messgenauigkeit noch weiter erhöht werden.

Das erfindungsgemäße Analyseverfahren weist die Merkmale des Anspruchs 8 auf.

Im Rahmen des Analyseverfahrens kann aus dem Bild des Lichtspalts eine Relativposition des Lichtspalts zu einer Referenzebene des Auges bestimmt werden. So kann vorgesehen sein eine Transversalebene des Auges als die Referenzebene zu definieren, wobei dann besonders einfach die Relativposition des Lichtspalts bzw. die sich daraus ergebenden Messergebnisse bestimmt werden können. Weiter kann die Referenzebene auch mit einer Referenzebene der ersten Analysevorrichtung übereinstimmen, wodurch eine Auswertung der Messergebnisse erheblich vereinfacht wird.

Aus dem Bild des Lichtspalts kann eine Tiefe t des Limbus relativ zu einem Apex in einer Sagittalebene des Auges bestimmt werden. Ein Maß der Tiefe t kann besonders gut zur Anpassung bzw. Auswahl einer Kontaktlinse herangezogen und weiter gegebenenfalls für eine Korrektur gemessener Krümmungsradien der Cornea verwendet werden. Demnach können die mit der ersten Analysevorrichtung gemessenen Topografiedaten mit der zweiten Analysevorrichtung korrigiert werden.

Ebenfalls zur Auswahl und Anpassung einer Kontaktlinse kann aus dem Bild des Lichtspalts ein Durchmesser d des Limbus bestimmt werden. Ein Maß des Durchmessers d ist, wie zuvor bereits ausgeführt, besonders wichtig für eine Auswahl eines Kontaktlinsendurchmessers.

Die Topografie des Limbus kann mittels Triangulation besonders genau berechnet werden. So kann beispielsweise durch den Einsatz der ersten Analysevorrichtung eine Relativposition des Analysegeräts bzw. ein Geräteabstand zu dem Auge bereits bekannt sein, so dass unter Nutzung dieser Daten die Topografie des Limbus aus dem betreffenden Bild des Lichtspalts abgeleitet werden kann.

Im Rahmen des Verfahrens kann es weiter vorgesehen sein, eine Mehrzahl von Bildern des Lichtspalts zu gewinnen, um gegebenenfalls eine erhöhte Messgenauigkeit zu erzielen.

Nachfolgend wird eine bevorzugte Ausführungsform der Erfindung der unter Bezugnahme auf die beigefügten Zeichnungen näher erläutert.

Es zeigen:
- **Fig. 1**: eine schematische Schnittansicht eines Analysegerätes mit einem Auge in einer Sagittalebene;
- **Fig. 2**: eine schematische Schnittansicht des Analysegerätes mit dem Auge in einer Transversalebene;
- **Fig. 3**: eine Vorderansicht des mit einer Projektionseinrichtung des Analysegeräts beleuchteten Auges;
- **Fig. 4**: eine schematische Schnittansicht eines weiteren nicht erfindungsgemäßen Analysegerätes mit einem Auge in einer Sagittalebene;
- **Fig. 5**: eine schematische Schnittansicht des weiteren Analysegerätes mit dem Auge in einer Transversalebene;
- **Fig. 6**: eine Vorderansicht des mit einer Projektionseinrichtung des weiteren Analysegeräts beleuchteten Auges.

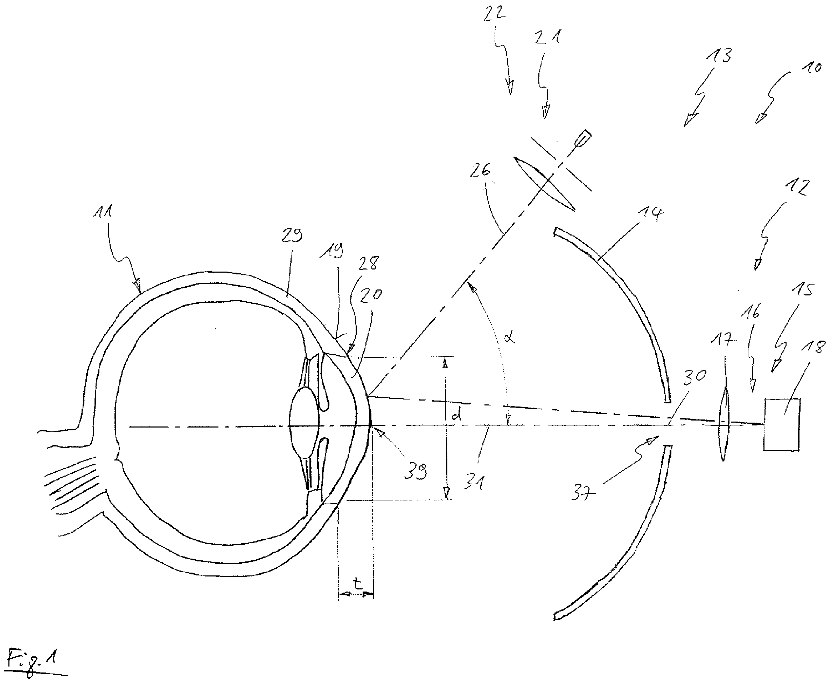

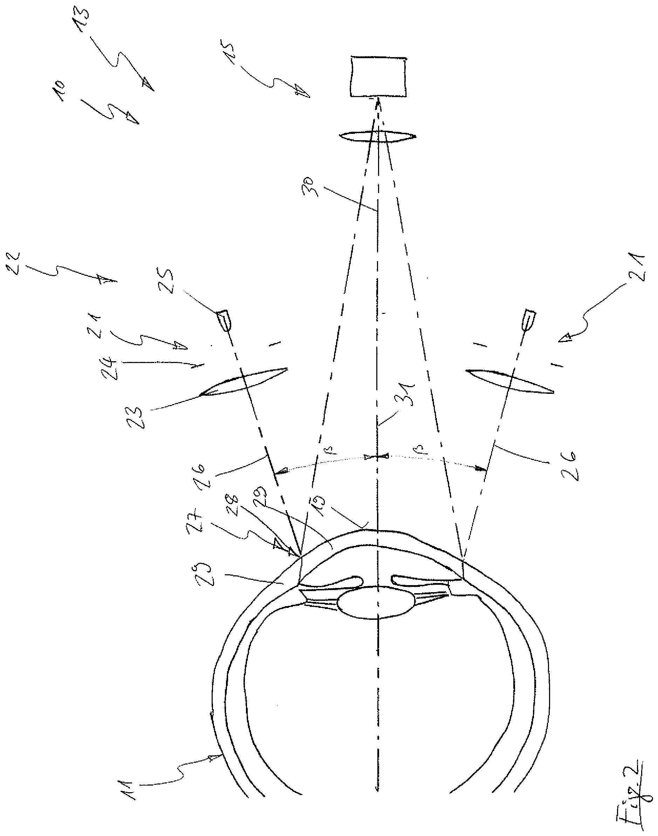

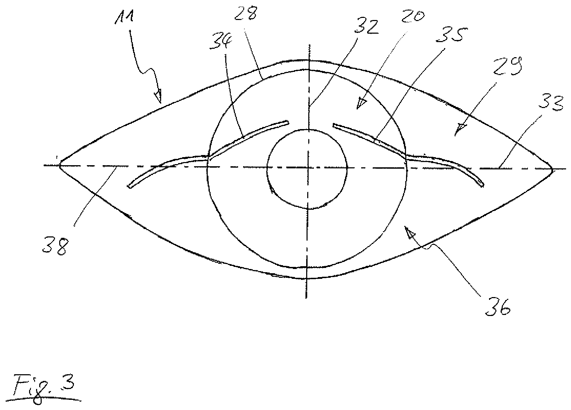

Eine Zusammenschau der **Fig. 1** und **2** zeigt eine schematische Darstellung eines Analysegerätes 10 zusammen mit einem Auge 11, welches in **Fig. 3** ergänzend in einer mit dem Analysegerät 10 beleuchteten Vorderansicht abgebildet ist. Das Analysegerät 10 ist aus einer ersten Analysevorrichtung 12 und einer zweiten Analysevorrichtung 13 gebildet. Die erste Analysevorrichtung 12 ist in Art eines Keratometers mit einer andeutungsweise dargestellten Placido-Projektionseinrichtung 14 und einer Beobachtungseinrichtung 15 bestehend aus einer Kamera 16 mit einem Objektiv 17 und einem Videosensor 18 ausgebildet. Mittels der Placido-Projektionseinrichtung 14 kann so ein Bildmuster konzentrischer Ringe auf eine Oberfläche 19 einer Cornea 20 des Auges 11 projiziert werden. Das Bildmuster kann dann mittels der Kamera 16 aufgenommen und mit einer hier nicht dargstellten Auswertevorrichtung so ausgewertet, dass eine Topografie der Cornea 20 bestimmt wird.

Die zweite Analysevorrichtung 13 umfasst die Beobachtungseinrichtung 15 der ersten Analysevorrichtung 12 und zwei Spaltprojektionseinheiten 21, welche eine Projektionseinrichtung 22 ausbilden. Die Spaltprojektionseinheiten 21 sind im Wesentlichen jeweils aus einem Objektiv 23, einer Spaltblende 24 und einer, eine Lichtquelle ausbildenden Leuchtdiode 25 gebildet. Die Spaltprojektionseinheiten 21 sind weiter so relativ zum Auge 11 angeordnet, dass ein Projektionsstrahlengang 26 der jeweiligen Spaltprojektionseinheit 21 auf einen Bereich 27 eines Limbus 28 zwischen der Cornea 20 und einer Sclera 29 des Auges 11 fällt. Der Projektionsstrahlengang 26 ist dabei relativ zu einer Geräteachse 30 der Beobachtungseinrichtung 15, welche fluchtend mit einer optischen Achse 31 des Auges 11 ausgerichtet ist, in einem Winkel angeordnet. So ist der Projektionsstrahlengang 26 in Bezug auf eine Sagittalebene 32 des Auges 11 in einem Winkel α und in Bezug auf eine Meridionalebene des Auges 11 in einem Winkel β angeordnet.

Wie aus **Fig. 3****,** welche eine Frontalansicht des Auges 11 aus Sicht der Beobachtungseinrichtung 15 zeigt, ersichtlich ist, werden jeweils Lichtspalte 34 und 35 der jeweiligen Spaltprojektionseinheiten 21 auf der Oberfläche 19 des Auges 11 abgebildet. Ein Bild 36 der Lichtspalte 34 und 35 wird dann mittels der Kamera 16 durch einen Durchlass 37 in der Placido-Projektionseinrichtung 14 hindurch aufgenommen. Die Auswertevorrichtung kann dann aus einer Position der Lichtspalte 34 und 35 relativ zu einer Referenzebene 38 sowie eine Gestalt der Lichtspalte 34 und 35 eine Krümmung der Cornea 20 und der Sclera 29 in dem Bereich 27 des Limbus 28 bestimmen. Weiter ist es leicht möglich einen Durchmesser d der Cornea 20 bzw. des Limbus 28 und eine Tiefe t des Limbus 28 relativ zu einem Apex 39 des Auges 11 zu berechnen.

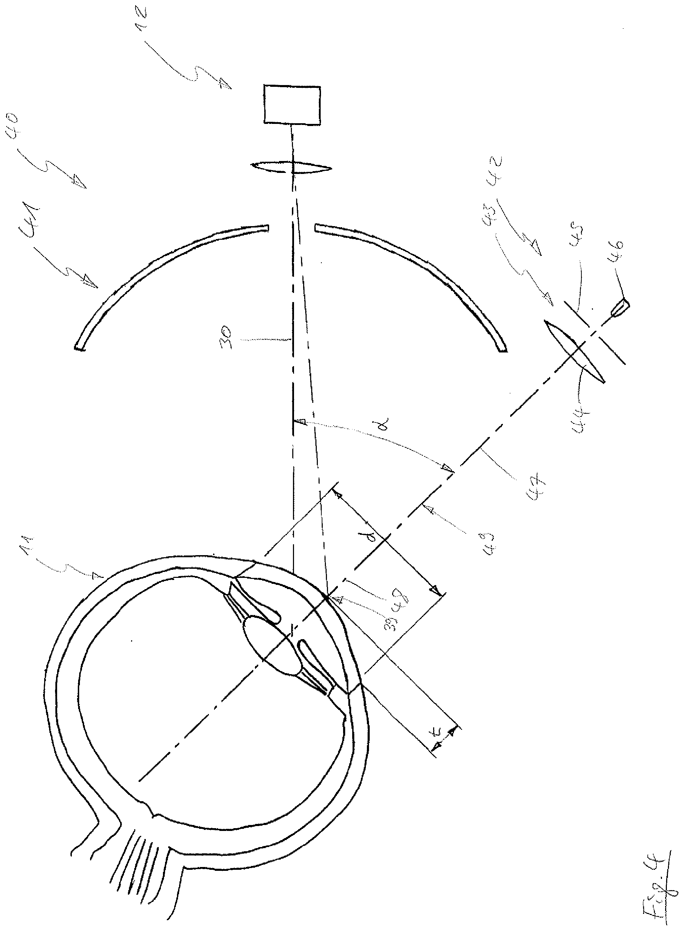

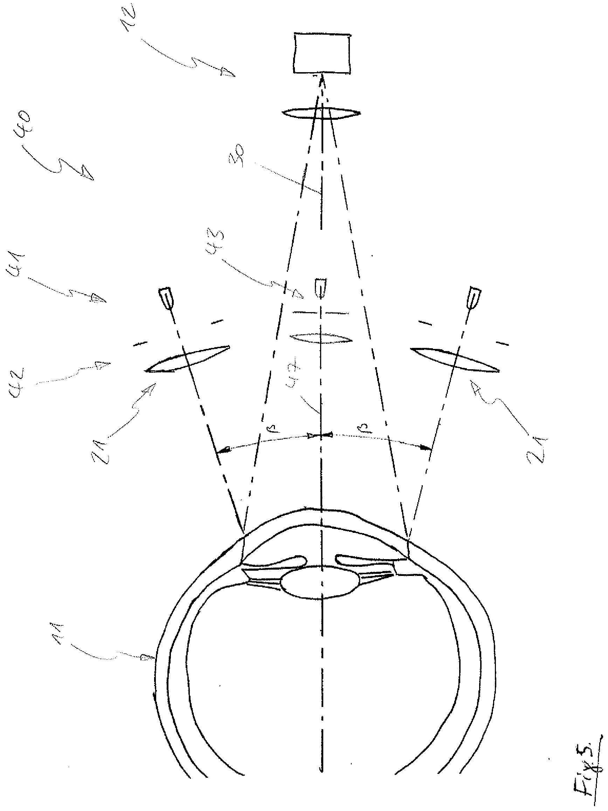

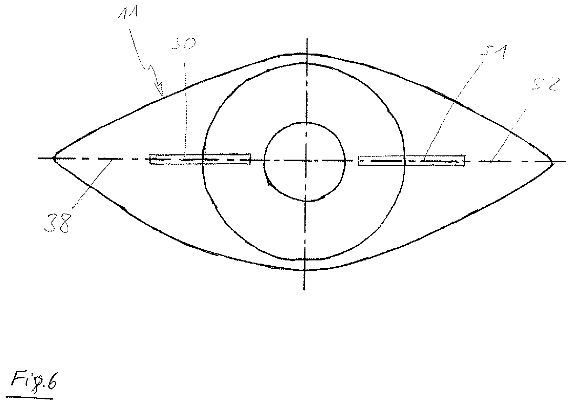

Eine Zusammenschau der **Fig. 4** und **5** zeigt eine schematische Darstellung eines weiteren Analysegeräts 40 zusammen mit dem Auge 11, welches in **Fig. 6** ergänzend in einer mit dem Analysegerät 40 beleuchteten Vorderansicht abgebildet ist. Das Analysegerät 40 umfasst ebenfalls die erste Analysevorrichtung 12 des zuvor beschriebenen Analysegeräts und eine zweite Analysevorrichtung 41. Die zweite Analysevorrichtung 41 weist die bekannten Spaltprojektionseinheiten 21 auf, welche eine Projektionseinrichtung 42 ausbilden. Die Projektionseinrichtung 42 umfasst hier zusätzlich eine Fixationseinheit 43, mit der eine Fixationsmarke darstellbar ist. Die Fixationseinheit 43 ist im Wesentlichen aus einem Objektiv 44, einer Blende 45 und einer eine Lichtquelle ausbildenden Leuchtdiode 46 gebildet.

Im Unterschied zu dem in **Fig. 1** und **2** beschriebenen Analysegerät ist beim Analysegerät 40 die Projektionseinrichtung 42 im Winkel α unterhalb der Geräteachse 30 angeordnet. Das Auge 11 wird hier so ausgerichtet, dass ein Fixationsstrahlengang 47 der Fixationseinheit 43 unmittelbar mit der optischen Achse 48 des Auges fluchtet bzw. auf diese ausgerichtet ist. Da die Spaltprojektionseinheiten 21 in einer gemeinsamen Spaltprojektionsebene 49 mit dem Fixationsstrahlengang 47 bzw. der optischen Achse 48 liegen, werden Lichtspalte 50 und 51 jeweils gerade und in einer Horizontalebene 52 des Auges 11, wie aus **Fig. 6** ersichtlich, abgebildet.

## Patentansprüche

1. Ophthalmologisches Analysegerät (10, 40) mit einer ersten Analysevorrichtung (12) zur Messung einer Krümmung einer Cornea (20) eines Auges (11), insbesondere Keratometer oder dergleichen, und mit einer zweiten Analysevorrichtung (13), umfassend eine Projektionseinrichtung (22) und eine Beobachtungseinrichtung (15), wobei die Beobachtungseinrichtung eine Kamera (16) umfasst, wobei die Projektionseinrichtung zwei Spaltprojektionseinheiten (21) zur Projektion jeweils eines Lichtspalts (34, 35, 50, 51) auf eine Oberfläche (19) des Auges umfasst, wobei mittels der Kamera ein Abbild (36) der Lichtspalte aufgenommen werden kann, wobei mit den Spaltprojektionseinheiten die Lichtspalte auf einen Bereich (27) eines Limbus (28) zwischen der Cornea und einer Sklera (29) des Auges projiziert werden können, wobei das Analysegerät eine Auswertevorrichtung aufweist, mittels der das Abbild analysiert werden kann, wobei aus dem Abbild der auf der Oberfläche abgebildeten Lichtspalte eine Topografie der Oberfläche in dem Bereich des Limbus abgeleitet werden kann, wobei mittels der Auswertevorrichtung aus einer Position und einer Gestalt der Lichtspalte eine Krümmung der Cornea und der Sklera im Bereich des Limbus bestimmt werden kann, wobei ein Strahlengang bzw. eine Geräteachse (30) der Beobachtungseinrichtung in einer Richtung einer optischen Achse (31) des Auges ausrichtbar ist, wobei ein Projektionsstrahlengang (26) der Spaltprojektionseinheiten relativ zum Strahlengang der Beobachtungseinrichtung in einem von dem Strahlengang abweichenden Winkel α angeordnet ist.

2. Analysegerät nach Anspruch 1,
**dadurch gekennzeichnet,**
**dass** der Projektionsstrahlengang (26) der Spaltprojektionseinheit (21) relativ zu einer Sagittalebene (32) in dem Winkel α und in einem von der Sagittalebene abweichenden Winkel β zu einer Meridionalebene (33) des Strahlengangs (30) der Beobachtungseinrichtung (15) angeordnet ist.

3. Analysegerät nach Anspruch 1 oder 2,
**dadurch gekennzeichnet,**
**dass** ein Durchlass (37) in einer weiteren Projektionseinrichtung (14) der ersten Analysevorrichtung (12) für den Strahlengang (30) der Beobachtungseinrichtung (15) ausgebildet ist.

4. Analysegerät nach einem der vorangehenden Ansprüche,
**dadurch gekennzeichnet,**
**dass** die Kamera (16) ein Objektiv (17) umfasst, wobei die Beobachtungseinrichtung (15) auch als eine Beobachtungseinrichtung der ersten Analysevorrichtung (12) dient.

5. Analysegerät nach einem der vorangehenden Ansprüche,
**dadurch gekennzeichnet,**
**dass** die erste Analysevorrichtung (12) ein Videokeratometer ist.

6. Analysegerät nach einem der vorangehenden Ansprüche,
**dadurch gekennzeichnet,**
**dass** die Spaltprojektionseinheiten (21) relativ zu einer Geräteachse (30) der Beobachtungseinrichtung (15) symmetrisch angeordnet sind.

7. Analysegerät nach einem der vorangehenden Ansprüche,
**dadurch gekennzeichnet,**
**dass** das Analysegerät (10) eine Auswertevorrichtung für beide Analysevorrichtungen (12, 13) aufweist, mittels der das Abbild (36) analysiert werden kann.

8. Analyseverfahren mit einem ophthalmologischen Analysegerät (10, 40) mit einer ersten Analysevorrichtung (12) zur Messung einer Krümmung einer Cornea (20) eines Auges (11), insbesondere Keratometer oder dergleichen, und mit einer zweiten Analysevorrichtung (13), umfassend eine Projektionseinrichtung (22) und eine Beobachtungseinrichtung (15), wobei die Beobachtungseinrichtung eine Kamera (16) umfasst, wobei die Projektionseinrichtung zwei Spaltprojektionseinheiten (21) umfasst, wobei mittels der Spaltprojektionseinheiten jeweils ein Lichtspalt (34, 35, 50, 51) auf eine Oberfläche (19) des Auges projiziert wird, wobei mittels der Kamera ein Abbild (36) der auf der Oberfläche abgebildeten Lichtspalte aufgenommen wird, wobei mit den Spaltprojektionseinheiten die Lichtspalte auf einen Bereich (27) eines Limbus (28) zwischen der Cornea und einer Sklera (29) des Auges projiziert werden, wobei das Analysegerät eine Auswertevorrichtung aufweist, mittels der das Abbild analysiert wird, wobei aus dem Abbild der auf der Oberfläche abgebildeten Lichtspalte eine Topografie der Oberfläche in dem Bereich des Limbus abgeleitet wird, wobei mittels der Auswertevorrichtung aus einer Position und einer Gestalt der Lichtspalte eine Krümmung der Cornea und der Sklera im Bereich des Limbus bestimmt wird ,wobei ein Strahlengang bzw. eine Geräteachse (30) der Beobachtungseinrichtung in einer Richtung einer optischen Achse (31) des Auges ausgerichtet wird, wobei ein Projektionsstrahlengang (26) der Spaltprojektionseinheiten relativ zum Strahlengang der Beobachtungseinrichtung in einem von dem Strahlengang abweichenden Winkel α angeordnet wird.

9. Analyseverfahren nach Anspruch 8,
**dadurch gekennzeichnet,**
**dass** aus dem Abbild (36) eine Relativposition des Lichtspalts (34, 35, 50, 51) zu einer Referenzebene (38) des Auges (11) bestimmt wird.

10. Analyseverfahren nach Anspruch 8 oder 9,
**dadurch gekennzeichnet,**
**dass** aus dem Abbild (36) eine Tiefe t des Limbus (28), relativ zu einem Apex (39) in einer Sagittalebene (32) des Auges (11) bestimmt wird.

11. Analyseverfahren nach einem der Ansprüche 8 bis 10,
**dadurch gekennzeichnet,**
**dass** aus dem Abbild (36) ein Durchmesser d des Limbus (28) bestimmt wird.

12. Analyseverfahren nach einem der Ansprüche 8 bis 11,
**dadurch gekennzeichnet,**
**dass** die Topografie des Limbus (28) mittels Triangulation berechnet wird.

## Claims

1. An ophthalmological analysis apparatus (10, 40) having a first analysis device (12) for measuring a curvature of a cornea (20) of an eye (11), in particular keratometers or the like, and having a second analysis device (13) comprising a projection device (22) and a monitoring device (15), wherein the monitoring device comprises a camera (16), wherein the projection device comprises two slit projection units (21) which each project a slit light (34, 35, 50, 51) onto a surface (19) of the eye, wherein an image (36) of the slit light can be recorded by means of the camera, wherein the slit lights can be projected onto an area (27) of a limbus (28) between the cornea and a sclera (29) of the eye by means of the slit projection units, wherein the analysis apparatus comprises an evaluation device by means of which the image can be analyzed, wherein a topography of the surface in the area of the limbus can be derived from the image of the slit lights projected onto the surface, wherein a curvature of the cornea and the sclera in the area of the limbus can be determined from a position and a shape of the slit lights by means of the evaluation device, wherein a beam path or an apparatus axis (30) of the monitoring device, respectively, can be adjusted in a direction of an optical axis (31) of the eye, wherein a projection beam path (26) of the slit projection units is arranged in relation to the beam path of the monitoring device in an angle α, which deviates from the beam path.

2. The analysis apparatus according to claim 1,
**characterized in that**
the projection beam path (26) of the slit projection unit (21) is arranged in relation to a sagittal plane (32) in the angle α and in relation to a meridional plane (33) of the beam path (30) of the monitoring device (15) in an angle β, which deviates from the sagittal plane.

3. The analysis apparatus according to claim 1 or 2,
**characterized in that**
a passage (37) is formed in a further projection device (14) of the first analysis device (12) for the beam path (30) of the monitoring device (15).

4. The analysis apparatus according to any one of the preceding claims,
**characterized in that**
the camera (16) comprises an objective (17), wherein the monitoring device (15) also serves as a monitoring device of the first analysis device (12).

5. The analysis apparatus according to any one of the preceding claims,
**characterized in that**
the first analysis device (12) is a video keratometer.

6. The analysis apparatus according to any one of the preceding claims,
**characterized in that**
the slit projection units (21) are arranged symmetrical in relation to an apparatus axis (30) of the monitoring device (15).

7. The analysis apparatus according to any one of the preceding claims,
**characterized in that**
the analysis apparatus (10) comprises an evaluation device for both analysis devices (12, 13), by means of which the image (36) can be analyzed.

8. An analysis method having an ophthalmological analysis apparatus (10, 40) having a first analysis device (12) for measuring a curvature of a cornea (20) of an eye (11), in particular keratometers or the like, and having a second analysis device (13) comprising a projection device (22) and a monitoring device (15), wherein the monitoring device comprises a camera (16), wherein the projection device comprises two slit projection units (21), wherein by means of each slit projection unit a slit light (34, 35, 50, 51) is projected onto a surface (19) of the eye, wherein an image (36) of the slit light projected onto the surface can be recorded by means of the camera, wherein the slit lights are projected onto an area (27) of a limbus (28) between the cornea and a sclera (29) of the eye by means of the slit projection units, wherein the analysis apparatus comprises an evaluation device by means of which the image is analyzed, wherein a topography of the surface in the area of the limbus is derived from the image of the slit lights projected onto the surface, wherein a curvature of the cornea and the sclera in the area of the limbus can be determined from a position and a shape of the slit lights by means of the evaluation device, wherein a beam path or an apparatus axis (30) of the monitoring device, respectively, is adjusted in a direction of an optical axis (31) of the eye, wherein a projection beam path (26) of the slit projection units is arranged in relation to the beam path of the monitoring device in an angle α, which deviates from the beam path.

9. The analysis method according to claim 8,
**characterized in that**
a relative position of the slit light (34, 35, 50, 51) to a reference plane (38) of the eye (11) is determined from the image (36).

10. The analysis method according to claim 8 or 9,
**characterized in that**
a depth t of the limbus (28) is determined from the image (36) in relation to an apex (39) in a sagittal plane (32) of the eye (11).

11. The analysis method according to any one of the claims 8 to 10,
**characterized in that**
a diameter d of the limbus (28) is determined from the image (36).

12. The analysis method according to any one of the claims 8 to 11,
**characterized in that**
the topography of the limbus (28) is calculated by means of triangulation.

## Revendications

1. Appareil d'analyse ophtalmologique (10, 40) ayant un premier dispositif d'analyse (12) pour mesurer une courbure d'une cornée (20) d'un oeil (11), en particulier des kératomètres ou similaire, et ayant un deuxième dispositif d'analyse (13) comprenant un dispositif de projection (22) et un dispositif d'observation (15), dans lequel le dispositif d'observation comprend une camera (16), dans lequel le dispositif de projection comprend deux unités de projection à fente (21) qui chacun projette une fente de lumière (34, 35, 50, 51) sur une surface de l'oeil, dans lequel une image (36) des fentes de lumière peut être enregistrée au moyen de la camera, dans lequel les fentes de lumière peuvent être projetées sur une partie (27) d'un limbe (28) entre la cornée et une sclère (29) de l'oeil au moyen des unités de projection à fente, dans lequel l'appareil d'analyse comprend un dispositif d'évaluation au moyen duquel l'image peut être analysée, dans lequel une topographie de la surface dans la partie du limbe peut être déduit de l'image des fentes de lumière projetées sur la surface, dans lequel une courbure de la cornée et de la sclère dans la partie du limbe peut être déterminée d'une position et d'une forme des fentes de lumière au moyen du dispositif d'évaluation, dans lequel une trajectoire du faisceau ou un axe d'appareil (30) du dispositif d'observation, respectivement, peut être ajusté(e) dans une direction d'un axe optique (31) de l'oeil, dans lequel une trajectoire du faisceau de projection (26) des unités de projection à fente est disposée relative à la trajectoire du faisceau du dispositif d'observation dans un angle α, qui diverge de la trajectoire du faisceau.

2. Appareil d'analyse selon la revendication 1,
**caractérisé en ce que**
la trajectoire du faisceau de projection (26) de l'unité de projection à fente (21) est disposée relative à un plan sagittal (32) dans un angle α et relative à un plan méridien (33) de la trajectoire du faisceau (30) du dispositif d'observation (15) dans un angle β, qui diverge du plan sagittal.

3. Appareil d'analyse selon la revendication 1 ou 2,
**caractérisé en ce qu'**
un passage (37) est formé dans un outre dispositif de projection (14) du premier dispositif d'analyse (12) pour la trajectoire du faisceau (30) du dispositif d'observation (15).

4. Appareil d'analyse selon l'une quelconque des revendications,
**caractérisé en ce que**
la camera (16) comprend un objectif (17), dans lequel le dispositif d'observation (15) sert aussi comme un dispositif d'observation du premier dispositif d'analyse (12).

5. Appareil d'analyse selon l'une quelconque des revendications,
**caractérisé en ce que**
le premier dispositif d'analyse (12) est un vidéo kératomètre.

6. Appareil d'analyse selon l'une quelconque des revendications,
**caractérisé en ce que**
les unités de projection à fentes (21) sont disposées symétrique relative à un axe d'appareil (30) du dispositif d'observation (15).

7. Appareil d'analyse selon l'une quelconque des revendications,
**caractérisé en ce que**
l'appareil d'analyse (10) comprend un dispositif d'évaluation pour les deux dispositifs d'analyse (12, 13) au moyen duquel l'image (36) peut être analysée.

8. Procédé d'analyse ayant un appareil d'analyse ophtalmologique (10, 40) ayant un premier dispositif d'analyse (12) pour mesurer une courbure d'une cornée (20) d'un oeil (11), en particulier des kératomètres ou similaire, et ayant un deuxième dispositif d'analyse (13) comprenant un dispositif de projection (22) et un dispositif d'observation (15), dans lequel le dispositif d'observation comprend une camera (16), dans lequel le dispositif de projection comprend deux unités de projection à fente (21), dans lequel chacun unité de projection à fente projette une fente de lumière (34, 35, 50, 51) sur une surface (19) de l'oeil, dans lequel une image (36) de la fente de lumière est enregistrée au moyen de la camera, dans lequel les fentes de lumière sont projetées sur une partie (27) d'un limbe (28) entre la cornée et la sclère (29) de l'oeil au moyen des unités de projection à fente, dans lequel l'appareil d'analyse comprend un dispositif d'évaluation au moyen duquel l'image est analysée, dans lequel une topographie de la surface dans la partie du limbe est déduite de l'image des fentes de lumière projetées sur la surface, dans lequel une courbure de la cornée et de la sclère dans la partie du limbe est déterminée d'une position et d'une forme des fentes de lumière au moyen du dispositif d'évaluation, dans lequel une trajectoire du faisceau ou un axe d'appareil (30) du dispositif d'observation, respectivement, est ajusté(e) dans une direction d'un axe optique (31) de l'oeil, dans lequel une trajectoire du faisceau de projection (26) des unités de projection à fente est disposée relative à la trajectoire du faisceau du dispositif d'observation dans un angle α, qui diverge de la trajectoire du faisceau.

9. Procédé d'analyse selon la revendication 8,
**caractérisé en ce qu'**
une position relative de la fente de lumière (34, 35, 50, 51) à un plan de référence (38) de l'oeil (11) est déterminée de l'image (36).

10. Procédé d'analyse selon la revendication 8 ou 9,
**caractérisé en ce qu'**
une profondeur t du limbe (28) est déterminée de l'image (36) relative à un apex (39) dans un plan sagittal (32) de l'oeil (11).

11. Procédé d'analyse selon l'une des revendications 8 à 10,
**caractérisé en ce qu'**
un diamètre d du limbe (28) est déterminé de l'image (36).

12. Procédé d'analyse selon l'une des revendications 8 à 11,
**caractérisé en ce que**
la topographie du limbe (28) est calculée au moyen de la triangulation.
